# EUROPEAN PATENT APPLICATION

(11) **EP 0 826 778 A1**
(43) Date of publication of application: **04.03.1998**
(21) Application number: 96202452.7
(22) Date of filing: 03.09.1996
(51) Int. Cl.: C12Q 1/68, C07H 21/04

(54) **Oligonucleotide probes for the detection of bifidobacteria**

(71) Applicant: Kaufmann, Peter, 6048 Horw (CH); Meile, Leo, 8103 Unterengstringen (CH); Teuber, Michael, 8044 Zürich (CH)
(72) Inventor: Kaufmann, Peter, 6048 Horw (CH); Meile, Leo, 8103 Unterengstringen (CH); Teuber, Michael, 8044 Zürich (CH)
(74) Representative: Arnold, Winfried

(57) **Abstract**

New oligonucleotides, their use for the rapid detection of bacteria of the genus *Bifidobacterium*, a method for the detection of bacteria of said genus, and an analytical kit for the qualitative and quantitative detection of said bacteria.

## Description

### Field of the Invention

The present invention concerns new oligonucleotides, their use for the rapid detection and quantification of bacteria of the genus *Bifidobacterium*, a method for the detection of bacteria of said genus, and an analytical kit for the qualitative and quantitative detection of said bacteria.

### Background of the Invention

Bifidobacteria are intestinal bacteria which play a major role in the most complex and diverse ecosystem of the intestinal tract of men as well as of warm blooded animals and honey bees (Biavati et al., 1991). Their role is still under investigation and numerous studies have been accomplished to investigate possible probiotic effects (Gibson and Robefriod, 1995; Grill et al., 1995; Lee and Salminen, 1995). In the food industry, bifidobacteria are widely used as food additives (Biavati et al., 1992; Ghoddusi and Robinson, 1996; Roy et al., 1995). However, it has been most difficult to monitor bifidobacteria counts in yoghurts, cheese, butter and other supposedly bifidobacteria-containing products since a truely selective medium which can discriminate between the genus *Bifidobacterium* and other genera does not exist up to now (Arroyo et al., 1995; Beerens, 1990; Lapierre et al., 1992). Discrimination of this genus by classical physiological and biochemical methods is often time consuming and takes a lot of effort and experience (Gavini et al., 1991).

Bifidobacteria are phylogenetically grouped in the Actinomycetes branch of Gram-positive bacteria (Schleifer and Ludwig, 1995; Sgorbati et al., 1995) containing a high G+C content (Olsen et al., 1994) and at present grouped into 32 species (11 species of human, 15 species of warm blooded animals, 3 species of honey bees, 2 species isolated from waste water and 1 from fermented milk, respectively) whose type strains are available at the DSM (Deutsche Sammlung von Mikroorganismen und Zellkulturen GmBH) culture collection.

In recent years, the attention of many taxonomically interested scientists has been drawn towards specific DNA probes (Amann et al., 1995; Bahaka et al., 1993; Mangin et al., 1995) and ribosomal RNAs, whose genes contain highly conserved regions and have the advantage of being found in a very large number within one single cell succeeding the number of chromosomally encoded genes 10,000 fold (Yamamoto et al., 1992) and thus being a prime target for an oligonucleotide probe. Many studies have been accomplished concerning 5S, 16S and 23S rRNA (Mangin et al., 1996; Ludwig and Schleifer, 1994; Stackebrandt and Goebel, 1994) as well as 16S to 23S rRNA internal transcribed spacer regions (Leblond-Bourget et al., 1996). Computational analysis of the specificity of 16S rRNA targeted oligonucleotides and probe hybridization have become worthy tools for the detection of various taxonomic features such as genera, species, and strains depending on the specificity of the probe (Brunk et al., 1996; Gendel, 1996). Various studies on bifidobacteria have shown 16S rRNA targeted probes which can detect different species (Frothingham et al., 1993; Yamamoto et al., 1992, or USP 5,443,951) or which detect the genus *Bifidobacterium* in the human fecal flora (Langendijk et al., 1995).

However, none of these probes is suitable for the detection of all strains of the genus *Bifidobacterium*, including industrially used strains or closely related species.

The polymerase chain reaction has been a promising method for detection of various bacterial species isolated from feces and food (Carrino and Lee, 1995; Rijpens et al., 1996). A recent study by Wang and coworkers (1996) showed a PCR based method for detection and quantification of anaerobic bacteria in human and animal fecal samples.

The results of the present invention have been presented on a poster during the 55. Annual Meeting of the Swiss Society for Microbiology from February 29 to March 1, 1996 in Bern, Switzerland, however, without disclosing the specific oligonucleotides of the present invention. Accordingly, the poster is a non-enabling disclosure.

There is a need for a full and enabling description of the new method.

### Object of the Invention

It is an object of the invention to provide a rapid and easy to handle, reliable, qualitative and quantitative method for the detection of all bacteria of the genus *Bifidobacterium*, such as isolated from food, feces and other samples. A further object is to provide a PCR-related technique for applications in samples, and to provide kits for the easy use of the present method in analytical laboratories for monitoring bifidobacteria counts in industrial, natural or environmental samples.

### Summary of the Invention

A useful tool for taxonomic and phylogenetic relationships is the comparison of the 16S rRNA especially from the level of domains to genus (Stackebrandt and Goebel, 1994; Frothingham et al., 1993). Less suitable is this method for the differentiation between different species, where DNA-DNA hybridization is more precise and reliable. The genus *Bifidobacterium* seems to be a closely related phylogenetic cluster, considering the evolutionary distances of 165 rRNA similarities of >93% (Leblond-Bourget et al., 1996). An EMBL/GenBank search revealed 30 partial or total 16S rRNA sequences from bifidobacteria. We aligned these sequences and selected a strech of 21 nucleotides (Table 3) which was highly conserved within the genus *Bifidobacterium* and which showed only one mismatch at the position 1224 (*E. coli* numbering, Brosius et al., 1981) containing either C or T. *Gardnerella vaginalis* was the sole representative among all bacteria which had a homologous sequence with a not determined nucleotide (N) at this position. The taxonomic positioning of this strain, which represents the only species of the genus *Gardnerella* is still in discussion. Embly and Stackebrandt (1994) assumed that *Gardnerella* and *Bifidobacterium* belong to the same genus. Other workers stated that *Gardnerella* and *Bifidobacterium* exhibit close phylogenetic proximity (Larsen et al., 1993) and that 16S rRNA similarity is consistent with placing *Gardnerella* and *Bifidobacterium* in the same genus (Leblond-Bourget et al., 1996). However, a separate genus-status of *Gardnerella* was established by comparing its low G+C content (42-44 mol% versus 55-67 mol% in bifidobacteria) (Biavatti et al., 1991) and on the basis of DNA-DNA hybridization experiments (Greenwood and Picket, 1986). Since DNA-DNA hybridization experiments are not suitable for the distinction of genera (Stackebrandt and Goebel, 1994), the question regarding the genus still remains to be answered. All other strains in the EMBLE/GenBank, however, had two or more mismatches within this sequence.

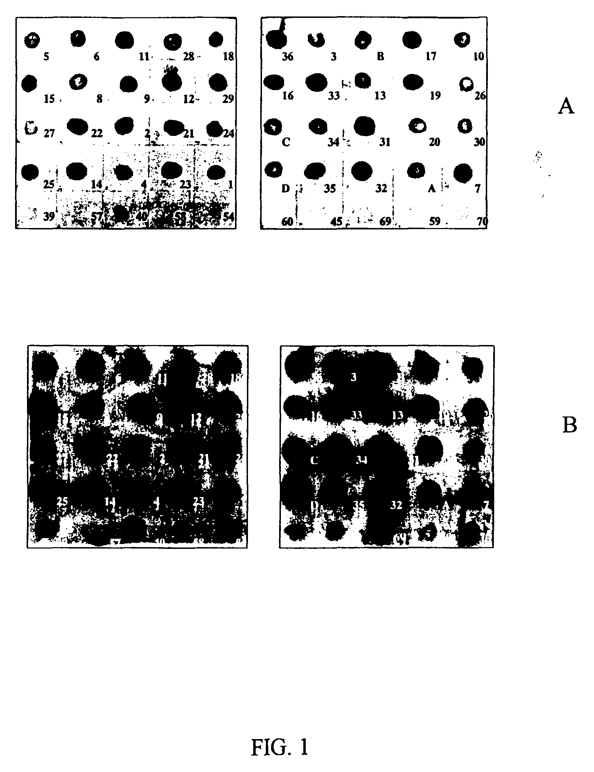

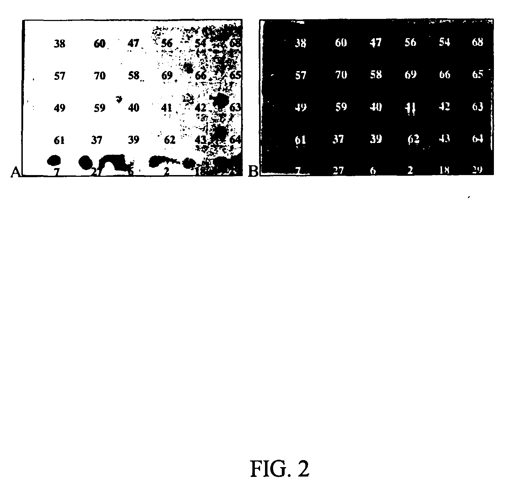

In order to approve the theoretically found specificity of the selected probe lm3 for *Bifidobacterium*, it had to be shown that this approach also functioned in empirical tests. Table 1 and Fig. 1 show colony-hybridization experiments with all bifidobacteria type strains available from the DSM culture collection except both recently published new species *Bifidobacterium inopinatum* and *Bifidobacterium denticolens* (Crociani et al., 1996). All of the *Bifidobacterium* type strains showed distinct signals in colony-hybridization experiments with the *Bifidobacterium* specific probe lm3. Negative controls including closely related bacteria and bacteria which are important in the dairy industry (Table 1 and Fig. 1 and 2) showed no signal after detection with probe lm3 and demonstrated the requested specificity. Weak signals can be seen for *Gardnerella vaginalis* which also meets our theoretical findings of only one mismatch in the target sequence (Table 3) and for *Propionibacterium freudenreichii* ssp. *shermanii*, whose 16S rRNA is only partially known (G. Dasen, personal communication). A weak signal could be detected with *Brevibacterium linens* but on the original membrane it was distinguished from the other dots by the reddish colour which originates from the pigment produced by this strain. Colony-hybridization experiments with [α-³² P]ATP labelled probe showed more distinct signals than with chemically labelled probe. This finding implies that radioactivity is still useful for this kind of difficult experiments despite the problem with its waste.

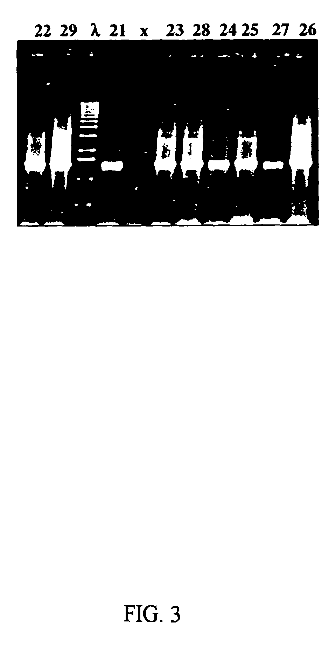

Another highly efficient way of detecting bifidobateria is the PCR-technique. Using the primers lm26 and lm3 (Table 2) we were able to amplify a segment of the 16S rRNA of the expected size of 1.35 kb in bifidobacteria but in no other bacteria tested (Table 1 and Fig. 3). Thus it is possible to rapidly and accurately distinguish bifidobacteria by means of PCR.

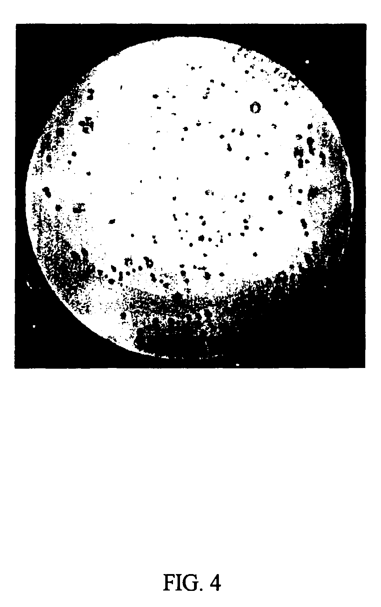

Isolation of bifidobacteria from food out of a complex bacterial flora was possible by plating serial dilution steps in semiselective agar-medium and incubating these agar plates in an anaerobe chamber. Colony counts of bifidobacteria was performed after plaque lift and colony-hybridization with the specific *Bifidobacterium* probe lm3 (Figure 4). We were able to find 2.1 x 10⁶ to 2.3 x 10⁷ cfu/g in commercially available dairy products.

### Detailed Description of the Invention

A *Bifidobacterium* genus-specific target sequence in the V9 variable region of the 16S rRNA was identified which was used to develop a hybridization probe. The specificity of this probe, named lm3 and having the sequence 5 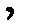 -CGGGTGCTNCCCACTTTCATG-3 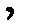 , Seq Id No 1, wherein N represents inosin, was used to identify all known type strains and distinguish them from other bacteria. All of the 30 strains of *Bifidobacterium* which are available at the DSM culture collection, six commercially available production strains and 42 closely related relevant strains as negative controls were tested. All tested bifidobacteria showed distinct positive signals in colony-hybridization whereas all negative controls showed no distinct dots except *Gardnerella vaginalis*, DSM4944, and *Propionibacterium freudenreichii* ssp. *shermanii*, DSM4902, which gave slight signals.

Furthermore, a method for isolation and identification of bifidobacteria from food has been established by a direct PCR assay without isolation of DNA comprising breaking the cells with Proteinase K whereby all *Bifidobacterium* strains lead to a DNA-product of the expected size.

Established was further a quick assay in order to quantitatively measure *Bifidobacterium* counts in food and feces by dilution plating and colony hybridization. The quantitative analytical detection of 2.1 x 10⁶ to 2.3 x 10⁷ colonies/g bifidobacteria containing sourmilk was demonstrated which hybridized with the specific nucleotide probe. With these two methods, genus specific colony hybridization and genus specific PCR, it is now possible to readily and accurately detect any bifidobacteria in food, fecal and any other samples and to discriminate them from other genera.

The invention further provides a method for the use of direct PCR for genus specific detection of bifidobacteria isolated from food without isolation of DNA.

In particular the present invention concerns a new oligonucleotide for the specific detection of bacteria of the genus *Bifidobacterium* comprising the sequence 5'-CGGGTGCTNCCCACTTTCATG-3', Seq Id No 1, wherein A,T,C and G have the meanings adenine, thymine, cytosine and guanine, respectively, and N represents inosine or another chemical hybridizing to all bases possibly present in the complementary position of the DNA or RNA bifidobacteria target, the complementary sequence thereof, a fragment thereof, or a labelled derivative thereof.

### Short description of the Figures

FIG. 1: Colony-hybridization of *Bifidobacterium* type strains with *Bifidobacterium* genus specific Dig-labelled probe lm3 (A) and with [α-³²P]ATP labelled universal probe Unip1 (B): first four lanes: *Bifidobacterium* type strains, last lane: negative controls. Numbers correspond to species listed in Table1. A-D *Bifidobacterium* sp. isolated from food.
FIG.2: Colony-hybridization of negative controls with *Bifidobacterium* genus specific Dig-labelled probe LM3 (A) and with universal probe Unip1 (B): First four rows: negative controls, last row: *Bifidobacterium* type strains. Numbers correspond to species listed in Table1. A-D *Bifidobacterium* sp. isolated from food.
FIG. 3: Agarose gel electrophoresis of PCR products with PCR primers lm26/lm3. Numbers correspond to strains in Table 1.
FIG. 4: Colony-hybridization of bifidobacteria after dilution-plating (10⁻⁵) from sourmilk to a nylon membrane. The probe was Dig-labelled lm3.

The oligonucleotide can be of the DNA (as in lm3) or RNA type, wherein thymine may be replaced by uracile or any other modified base-analogon.

Another chemical hybridizing to the regular bases C, T or N present in the corresponding target sequence of *Bifidobacterium* ssp. (Table 3) is for example inosin or xanthosine which, like inosine, matches all four naturally occurring bases.

The letter N in the target sequence signifies a base not yet identified and can be any of the four regular bases of DNA or RNA.

The *Bifidobacterium* target site is single stranded and is located between bp 1412 and 1432 (E. coli numbering, Brosius et al., 1981) in the V9 variable region of the 16S rRNA of bifidobacteria. Its complementary sequence is 5' CATGAAAGTGGGNAGCACCCG-3', Seq Id No 1,wherein N is not determined for certain *Bifidobacterium ssp*, or is mostly C or T (Table 3).

The complementary sequence of Seq Id No 1, i. e. its target site, can also be used as a probe.

A hybridizing fragment has down to 13 or even only 7 nucleotides of Sequ Id No 1; and which is able to selectively detect bacteria of the genus Bifidobacterium. It is also useful as a primer in a PCR reaction.

The invention concerns in particular an oligonucleotide having the sequence 5'-CGGGTGCTNCCCACTTTCATG-3', Seq Id No 1, wherein N represents inosine. This oligonucleotide is named lm3.

For easy detection an oligonucleotide according to the invention is labelled at one or several nucleotides, e. g. by an enzymatic, chemifluorescent, luminescent or radioactive label. Said labels are detectable after hybridization with the target by the appropriate analytical method. Labelled derivatives of the present oligonucleotides are such with conventional lables, for example with chemical reporter groups, e. g. digoxigenine (Dig) or biotin, fluorescing groups, e. g. nitrobenzofuran, or enzymes, e. g. alcaline phosphatase or peroxidase, or with radioactive isotopes, e. g. ³²P, ³H, ³⁵S or ¹²⁵I. The label is a single or multiple one and is located at the 5' or 3' end or anywhere in the middle of the probe. 5' end labelling is accomplished e. g. by attaching γ-³²P or γ-³⁵P of an ATP to the hydroxy group of the DNA by means of T4 polynucleotide kinase. Terminal transferase is used to attach one or more nucleotides, e. g. α-³²P- or α-³⁵P-dATP or digoxigenine-dUTP, to the free 3'-hydroxy group of the probe.

The oligonucleotides and their labelled derivatives are prepared according to conventional methods, e. g. by means of an automated DNA synthesizer, followed by appropriate labelling or by using labelled nucleotides during the synthesis.

Detection of the labelled probe on the target is accomplished by the appropriate method known in the art.

Preferably the oligonucleotide probe is labelled at one or several positions with digoxigenin (Dig) or [α-³²P]ATP and detected by an antibody/antigen reaction, e. g. by means of the commercially available "Dig nucleic acid detection kit" of Boehringer-Mannheim, Germany, or by radioactivity measurement, e.g. with an x-ray-film or any other device, respectively.

Hybridizing means formation of hydrogen bonds between complementary base pairs of the single stranded probe and the single stranded target to give a stable double stranded hybrid nucleotide. The hybridization conditions are of conventional manner.

The hybridization process is dependent on the number of matching base pairs, the number of Gs and Cs in the sequence, the pH of the medium and eventually the dimethyl formamide concentration, as is wellknown in the art.

The invention concerns further the use of an oligonucleotide according to the present invention for the detection of bacteria of the genus *Bifidobacterium*. How the present oligonucleotides are used is in more detail described further below.

The invention concerns further a method for the detection of bacteria of the genus *Bifidobacterium* comprising targeting an oligonucleotide according to the invention to the 16S rDNA and/or 16S rRNA target site of said bacteria.

In connection with the present use and method the term detection comprises the qualitative and quantitative identification and determination of the envisioned bacteria. The qualitative identification and determination is achievied with living or dead bacteria. The quantitative method is preferably achieved with living bacteria.

The method comprises the isolation of bifidobacteria from food or other sources in that a sample is aerobically collected, suspended in a proper non-selective or preferably selective or semi-selective growth medium, e. g. in Bif-medium which is a non-selective Brain-Heart infusion medium (Biolife, Italy) supplemented with yeast extract, L-cystein hydrochloride and resazurin, or in the semi-selective modified LP-medium according to Lapierre et al., 1992, containing also L-cystein hydrochloride and nalidixic acid, and incubation under anaerobic conditions preferably at 37 °C. The gas atmosphere is oxygen free and contains for example about 94% nitrogen and 6% hydrogen. It can be kept free of oxygen by addition of a commercially available oxygen binding chemical and of carbon dioxide.

After anaerobic incubation for quantitave determination serial dilution may be performed on agar plates, e. g. LP-agar containing nalidixic acid and anaerobic incubation performed as described above. The colonies are either harvested, resuspended in Bif-medium and transfered to a proper membrane, such as a nylon or nitrocellulose membrane, or directly transfered, e. g. by plaque lifts, onto such membrane.

The bacteria are pretreated in order to set free the target DNA and/or RNA in single stranded form. For this purpose the cells are lysed with lysozyme, e. g. on 3MM paper filters in the presence of Tris-HCl and succrose at pH 7.5 and incubating for about one hour at 37 °C. The DNA and RNA is denaturated, e. g. with sodium chloride solution and neutralized, e. g. with Tris-HCl, pH8, and washed with SSC. After drying the DNA is fixed to the membrane, e. g. by UV-crosslinking.

Prehybridization is performed in a suitable, commercially available prehybridization buffer (e. g. Sambrook et al., 1989) and hybridization with a labelled oligonucleotide of the present invention, e. g. with digoxigenine- or [α-³²P]ATP labelled lm3, at about 55 -60 °C, preferably at 57 °C in a suitable hybridization buffer (e. g. Sambrook et al., 1989), followed by detection of the labelled and hybridized oligonucleotide. Detection of the labelled oligonucleotide is performed according to conventional methods known in the art, such as a colour or chemiluminiscent reaction or measurement of the radioactivity.

Quantification of the number of bifidobacteria in a sample is achieved by enumeration of the viable bifidobacteria colonies after serial dilution plating and counting of the positive signals on the membranes after hybridization of the colony lifts.

An alternative method for the detection of bifidobacteria comprises application of the PCR technique. The method is particular useful if only a small amount of a sample is available. The bacteria are lysed, eventually after growing them under anaerobic conditions as described above, by applying a digestion buffer, e. g. consisting of Tris-HCl, EDTA, SDS and Proteinase K, for about 3 hours at about 55 °C. The Proteinase K is deactivated at about 95 °C for about 10 minutes and the cell debris is removed, e. g. by centrifugation. The supernatant comprising the nucleic acids of the bifidobacteria is subjected to a PCR according to a conventional method, e. g. applying 35 cycles with the primers lm 26, Seq Id No 2, and lm3, Seq Id No 1, wherein N is inosine, and a suitable DNA polymerase, e. g. Taq polymerase. The amplified nucleic acids are separated, e. g. by horizontal electrophoresis on agarose gel, stained with ethidium bromide and visualized with UV-light.

In another method, in particular useful if bacteria are available in high density, the DNA is extracted and restricted with restriction enzymes into small fragments. The fragments are subjected to electrophorese and transfered onto filters for Southern blot analysis. The probe is hybridized to the denatured DNA fragments on the filter and the hybridizing fragments are visualized by a conventional method.

The present method according to the invention is preferably such in which the detection is based on a polymerase chain reaction technique by means of an oligonucleotide according to the invention, whereby in addition a primer comprising the sequence 5'-GATTCTGGCTCAGGATGAACG-3', named lm26, Seq Id No 2, or a hybridizing partial sequence thereof is applied.

In another preferred method the detection is based on a dot-blot assay, where the DNA or RNA of the sample is fixed on a nylon membrane.

In another method according to the invention, which is particularly useful for quantitative detection, the bacteria are serially diluted and streaked out on agar-plates.

In particular the methods according to the invention are used to detect bacteria of the genus *Bifidobacterium* in food, feces or any other environment. Bifidobacteria are in particular present in food stuff such as dairy products which are often supplemented therewith, e. g. in milk drinks, soft cheese, butter, or yoghurt.

The invention concerns further analytical kits, useful for the qualitative and quantitative detection of bacteria of the genus *Bifidobacterium*. Such kits comprise at least an oligonucleotide according to the present invention, optionally a second oligonucleotide primer as described hereinbefore and optionally other conventional materials needed for the qualitative or quantitative detection and identification of bacteria of the genus *Bifidobacterium*.

Such materials are for example, oligonucleotide lm3, oligonucleotide lm26, digoxigenin labelled lm3, universal probe Unip1, nylon membranes, 3MM paper filters, Tris/HCl, saccharose, lysozyme, Wincam densitometry package, 16S rRNA from a bifidobacterium as positive probe, Proteinase K, SDS, digestion buffer, a DNA polymerase, and agarose gels.

The following examples serve to further illustrate the invention, however, they should not be construed as a limitation thereof.
The following abbreviations are used:
- EMBL: European Molecular Biology Laboratory Data Library
- cfu: colony forming units
- EDTA: ethylenediaminetetraacetic acid
- PCR: polymerase chain reaction
- SDS: sodiumdodecylsulfate
- SSC: sodium chloride and sodium citrate: 3 M NaCl, 0,3 M Na-citrate, pH 7.4 (standard buffer solution)
- Tris-HCl: tris(hydroxymethyl)aminomethane hydrochlorid, containing HCl to adjust to various pH values

### Example 1. Bacterial strains and growth conditions.

All bacterial strains used in this study are listed in Table 1. *Bifidobacterium* strains were grown anaerobically at 37°C either in non-selective Bif-medium (Brain-Heart Infusion (37 g/l), Biolife, supplemented with yeast extract (5 g/l), L-cystein-hydrochloride (0.5 g/l) and resazurin (2 mg/l)) or in semi-selective LP-medium (Lapierre et al., 1992), also containing 0.5 g/l cysteine hydrochloride and nalidixic-acid (30 mg/l) where indicated. Preparation of media and growth experiments involving *Bifidobacterium* strains on agar plates were carried out in an anaerobic chamber (COY Laboratories Products Inc., Ann Arbor, MI, USA) at a gas athmosphere of 94% nitrogen and 6% hydrogen. Agar-plates were incubated in anaerobic jars which were supplemented with a pad of Anaerocult C (Merck) that generates an oxygen-depleted and CO₂-enriched atmosphere. Incubation period took between 10 hours and three days depending on the strain. A stock of strains was kept at -72°C in Bif-medium containing 43% glycerol. For colony-hybridization experiments, frozen cultures were thawed on ice, inoculated in 10 ml of either Bif- or LP-medium and grown untill they reached the end of the logarithmic growth phase (optical density at 600 nm ∼ 0.9).

### Example 2. Isolation of bifidobacteria from food.

Dairy products supposedly containing bifidobacteria were purchased in drug-stores mainly in Switzerland, but also in Germany and France. Products consisted of yoghurt, soft cheese, butter and 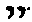 Bifidus-drink 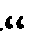 , a kind of milk drink supplemented with bifidobacteria. 10 g of a food sample were aerobically taken and dissolved in 90 ml of reduced (cysteine containing) Bif-medium, mixed until a homogenous suspension was obtained and quickly brought into the anaerobic chamber. Serial dilutions were then performed and streaked out onto LP-agar containing 30 mg/l of nalidixic acid. After anaerobic incubation as described above, agar-plates were taken out of the anaerobic chamber and colony hybridization was performed as will be described below.

### Example 3. Colony hybridization with Digoxigenin-labelled probe.

Colonies were transferred onto a nylon membrane (Hybond-N, nylon membrane, Amersham, Life science) either by harvesting 500 µl of an end log-phase culture (see above) and pipetting 10 µl of the resuspended pellet (in Bif-medium) onto the membrane or by direct plaque lifts as described by the supplier (Amersham, Life science). As next steps, DNA had to be liberated, neutralized, washed and fixed to the membrane. This was achieved by a series of solution-saturated 3MM paper filters. First, lysis of the cells was performed on saturated 3MM paper (10 mM Tris-HCl, 250 mM sucrose, and 5 mg/ml lysozyme, pH 7.5) by incubating the membrane for 60 min at 37°C. After this, each step was performed for 4 min on saturated 3MM paper with the following solutions: denaturation-solution (1 M NaCl), neutralization-solution (1 M Tris-HCl, pH 8), and washing-solution (2 x SSC). Nylon-membranes were dryed at 80°C during 10 min and DNA was fixed to the membrane by UV-crosslinking (4 min, at 302 nm).

The oligonucleotide lm3 was 3'-endlabelled with digoxigenine by means of the "Dig-oligonucleotide labelling kit" as described by the manufacturer (Boehringer-Mannheim, Germany).

Prehybridization was performed in a prehybridization buffer (5 x SSC, 1 x Denhardt's reagent, 50 mM Na-phophate, 250 µg/ml denaturated, fragmented salmon sperm DNA (Sambrook et al. 1989) in a total of 30 ml). Hybridization was performed in a hybridization buffer (5 x SSC, 1 x Denhardt's reagent, 40 mM Na-phophate, 500 µg/ml denaturated, fragmented salmon sperm DNA (Sambrook et al. 1989) and in addition containing 3 pmol/ml digoxigenin-labelled probe lm3 in a total of 30 ml). The reaction was performed at 57°C. Detection of the Dig-labelled DNA-DNA or DNA-RNA hybrids was performed with a 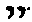 Dig nucleic acid detection kit 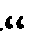 (Boehringer Mannheim, Germany) as described by the manufacturer. Evaluation of the dots on the membranes was accomplished by scanning the membranes with a video copy processor (Mitsubishi) and analysing these data with the Wincam densitometry package (Cybertech, Germany).

### Example 4. Colony hybridization with [α-³²P]ATP labelled probe.

Nylon membranes were prepared as described above and after hybridization with the bifidobacteria-specific probe they were stripped as described by the manufacturer (Boehringer-Mannheim, Germany). DNA probes (approx. 0.5 µg) for radioactive hybridization were labelled with 40-50 µCi [α-³²P]ATP (3 x 10³ Ci/mmol) using the 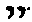 random priming 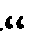 technique of Feinberg and Vogelstein (1983). Prehybridization and hybridization with [α-³²P]ATP labelled probe, in the appropriate buffers as described above, were done at 65°C in a Micro-4 hybridization oven (Hybaid). The membrane filter was then washed and subjected to autoradiography according to Sambrook et al. (1989).

### Example 5. Quantification of viable bifidobacteria.

Viable bifidobacteria were enumerated after serial dilution-plating, incubation and hybridization of colony-lifts by counting the number of positive signals on the membrane.

### Example 6. Selection of target-sequences for colony-hybridization.

Total 16S rRNA sequences of 20 *Bifidobacterium* ssp. and 23 partial 16S rRNA sequences were retrieved from the EMBL and GenBank data libraries (Benson et al., 1993; Neefs et al., 1993; Rice et al., 1993). Multiple alignments with all the bifidobacteria strains were performed and with these findings two target regions, lm3 and lm26, were chosen for PCR primers and *Bifidobacterium* ssp. specific oligonucleotide probes (Table 2). The oligonucleotide lm3 showed one or no missmatches with all available 16S rDNA sequences from *Bifidobacterium* ssp., which is a prerequisite for a genus-specific probe. Mismatches were due to unknown bases in the sequence.

These two probes were compared to all available bacterial data in the two gene banks stated above.

### Example 7. Polymerase chain reaction.

Fast DNA extraction from bifidobacteria was performed by a modified procedure statet earlier (Goldenberger et al., 1995) using proteinase K and SDS. The procedure was as follows: 10 ml of Bif-medium was inoculated with a single colony and grown under anaerobic conditions to a cell number of 10⁸ cfu/ml. 0.5 ml were harvested and diluted in 0.2 ml digestion buffer ( 50 mM Tris-HCl, pH8; 1 mM EDTA; 0.5% SDS; 1 mg/ml Proteinase K). After incubation at 55°C for 3 h Proteinase K was inactivated at 95°C for 10 min and cell debris was removed by centrifugation. 10 µl of the supernatant were applied for the polymerase chain reaction (PCR), adding 2% Tween 20 (final concentration) using the Dynazyme™ polymerase II F-501L polymerase (Finnzymes Oy, Finland). A 50 µl amplification mix contained the following chemicals: sample of the above test solution (10 µl), optimized Dynazym buffer (Finnzymes Oy, Finland), polymerase (2 units), dNTPs (300 µM, primer pair (1µM each). PCR parameters in the Perking Elmer thermal cycler were the following: Denaturation: 94°C, 1 minute, annealing: 57°C, 3 min, elongation: 72°C, 4 min. After 35 cycles the reaction was cooled down to 4°C or frozen at -20°C until further use. DNA was fractionated by horizontal electrophoresis on 0,8% agarose gels and stained with ethidium-bromide.

### Example 8. Test Kit for colony or dot-blot hybridizations (40 tests)

A test kit for the qualitative or quantitative determination of bacteria of the genus *Bifidobacterium* comprises the materials used in the preceding Examples, in particular

| Solution | Description | dilution factor | amount |
|---|---|---|---|
| S1 | lysis solution | 100x | 4 ml |
| | Lysozyme | | 2 g |
| S2 | denaturation solution | 100x | 4 ml |
| S3 | nutralization solution | 100x | 4 ml |
| S4 | washing solution | 100x | 4 ml |
| H1 | prehybridization solution | 10x | 45 ml |
| H2 | hybridization solution | 10x | 42 ml |
| H3 | washing solution | 10x | 105 ml |
| lm3 | Dig-labelled oligonucleotide lm3 solution | 0.3 pmol/µl | 400 µl |
| unip 1 | Dig-labelled positive probe solution | 0.3 pmol/µl | 400 µl |
| P1 | positive control for dot blots: DNA of *B. lactis,* solution | 1 pmol/µl | 400 µl |
| P2 | positive control for colony-hybridizations: viable cells of *B. lactis* | | 1 g |

The test kit comprises further a description of how to perform the test.

### Example 9. Test Kit for PCR detection of bifidobacteria (100 samples)

A test kit for the qualitative or quantitative determination of bacteria of the genus *Bifidobacterium* comprises the materials used in the preceding Examples, in particular

| Solution | Description | dilution factor | amount |
|---|---|---|---|
| 1 | Digestion buffer | 100x | 20 ml |
| | Proteinase K | | 4 g |
| 2 | Tween 20 | 10% | 1 ml |
| 3 | polymerase buffer | 10x | 500 µl |
| 4 | dNTP 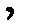 s (dATP, dCTP, dGTP and dTTP) solution | 300 µM each | 750 µl |
| 5 | primer lm3 solutiom | 1 µM | 100 µl |
| 6 | primer lm26 solution | 1 µM | 100 µl |
| 7 | polymerase solution | 2200 units | 100 µl |
| 8 | positive control: DNA of *B. lactis* solution | 1 µg | 100 µl |

The test kit comprises further a description of how to perform the test.

### Discussion of the Results

The results obtained in the described Examples are discussed in the following: **Genus-specificity of selected target region.** The proposed target region in the 16S rRNA gene for *Bifidobacterium* genus specific probe lm3 (Table 2) was aligned with all bacterial sequences obtained from the EMBL/GenBank. This alignment revealed that the 16S *rrna* genes of all the Bifidobacterium species showed no mismatch with the lm3 sequence (Table 3). The position with Inosine (N = I in lm3) is covered by a Cytosine (C) in the 16S rRNA sequences of 22 subspecies belonging to 8 species whereas a Thymine (T) takes this place among 9 subspecies belonging to 7 species. However, the 16S rDNA sequence of *Bifidobacterium angulatum* showed an ambiguity at the first position of the target sequence. *Bifidobacterium dentium* and *Gardnerella vaginalis* had an ambiguity at the Inosine site of the primer. All other bacteria including lactic acid bacteria had two or more mismatches at the position of the target site.
**Colony-hybridizations with *Bifidobacterium* specific probe.** After colony hybridization and colorimetric detection with the Dig-nucleic acid detection kit, typical images as shown in Fig. 1 and 2 were obtained by a video camera. All of the *Bifidobacterium* type strains and all of the *Bifidobacterium* production strains listed in Table 1 showed distinct dots after hybridization with the genus specific probe lm3, whereas all controll-strains did not hybridize with the specific probe lm3 (Table 1 and Fig. 1A), the bacteria showed dots with the universal probe Unip1 (Fig. 1B). The genus-specificity of probe lm3 was further investigated for bacterial species which are closely related to *Bifidobacterium* (Fig. 2). In colony-hybridizations no distinct dots can be seen except at the position of *Gardnerella vaginalis* and *Propionibacterium freudenreichii* sp. *shermanii* on the membrane (Fig. 2A) which showed weak signals. In order to prove that there was actually DNA at the dots of the negative controls, the universal probe Unip1 (Table 2) which detects all DNA was used as hybridization probe. The results of these hybridization experiments are presented in Fig. 1B and 2B. Dots in all the positions mean the presence of DNA in all the bacterial samples.
**Polymerase chain reaction of bifidobacterial 16S rDNA.** Another method to detect and identify bifidobacteria was done by polymerase chain reaction (PCR) using lm26 and lm3 as primers (Table 2). The target-DNA for these primers was obtained by a simple lysis procedure (see Materials and Methods). An example of this PCR-approach is shown in Fig. 3. In additon, representatives of all *Bifidobacterium* species produced a distinct band at a size of 1.35 kb, which represents the size of the 16S rDNA fragment amplified by the PCR whereas bacteria other than bifidobacteria gave no band at all (data not shown).
**Quantification of viable bifidobacteria isolated from food.** After dilution plating of different food samples (cheeses, yohurts, butter and 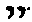 probiotic 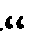 drinks containing bifidobacteria), colony-lift and hybridization with lm3, distinct dots were observed on the membrane representing colonies of bifidobacteria (Fig. 4). These colonies could be counted manually and colony forming units per gram food sample or feces were calculated. Analysing 8 different sourmilk products we were able to find 2.1 x 10⁶ to 2.3 x 10⁷ cfu/g bifidobacteria which hybridized with the specific nucleotide probe. Yoghurt without bifidobacteria showed no signals after hybridization (data not shown). Analysing human feces we detected 10⁸ cfu/g bifidobacteria (data not shown). These findings demonstrated the suitability of this method.

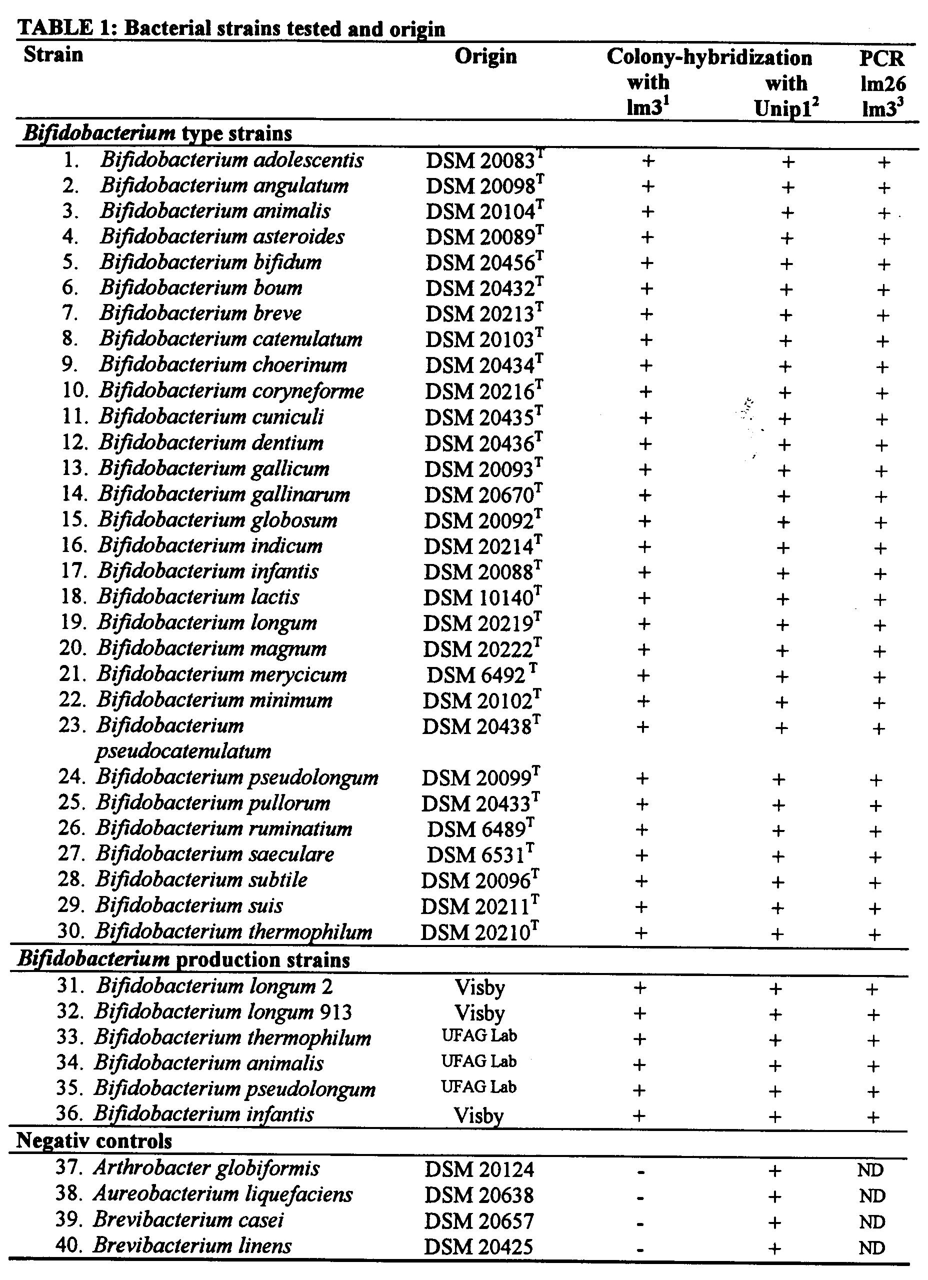

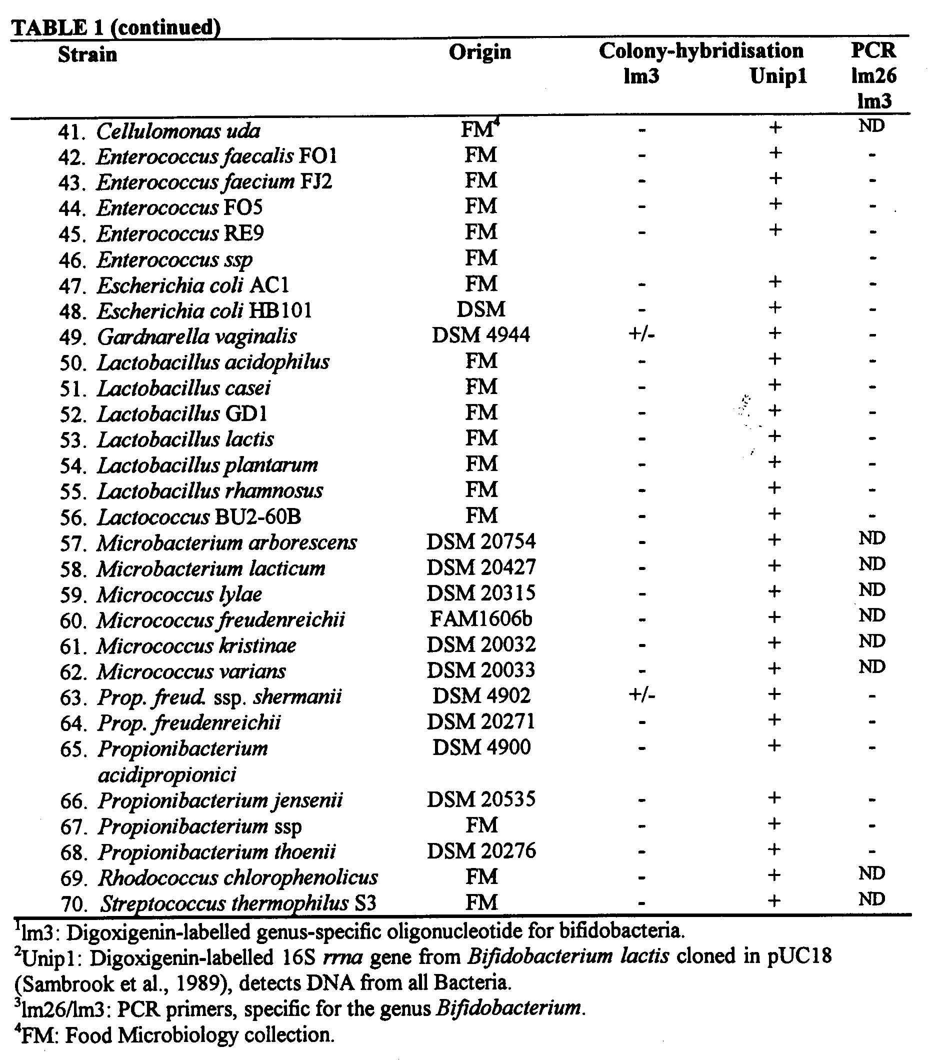

**TABLE 2**

| **Sequences, hybridization and washing temperatures of the different probes** | | | | |
|---|---|---|---|---|
| Probe | Sequence | Direction | Target site¹ | Temperature (°C) for Hybridization and Washing |
| lm26 | 5 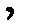 -GATTCTGGCTCAGGATGAACG-3 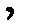 | forward | 15-35 | 57 |
| lm3³ | 5 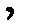 -CGGGTGCTICCCACTTTCATG-3 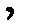 | reverse | 1412-1432 | 57 |
| Unip1² | 16S *rrna* gene of *Bifidobacterium lactis* | | | 65 |

| | | | | |
|---|---|---|---|---|
| ¹Corresponds to *E. coli* numbering of 16S rRNA gene (Brosius et al., 1981) | | | | |
| ²See remark in Table 1. | | | | |
| ³I: Inosine, matches all four nucleotides. | | | | |

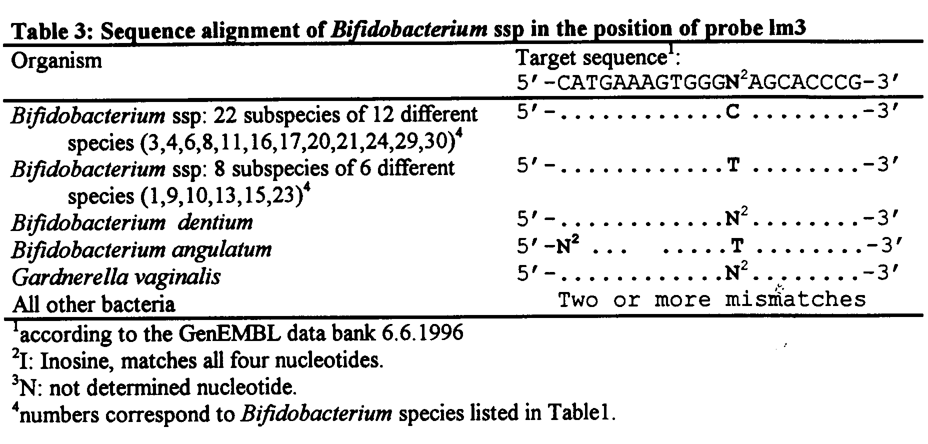

### References

1. Amann, R. I., W. Ludwig, and K. H. Schleifer. 1995. Phylogenetic identification and *in situ* detection of individual microbial cell without cultivation. Microbiol. Rev. **59:**143-169.
2. Arroyo, L., L. N. Cotton, and J. H. Martin. 1995. AMC agar- a composite medium for selective enumeration of *Bifidobacterium longum*. Cult. Dairy Prod. J. **2:**12-15.
3. Bahaka, D., C. Neut, A. Khattabi, D. Monget, and F. Gavini. 1993. Phenotypic and genomic analysis of human strains belonging or related to *Bifidobacterium longum*, *Bifidobacterium infantis*, and *Bifidobacterium* breve. Int. J. Syst. Bacteriol. **43:**565-573.
4. Beerens, H. 1990. An elective and selective isolation medium for *Bifidobacterium* ssp. Lett. Appl. Microbiol. **11:**155-157.
5. Benson, D., D. J. Lipman, and J. Ostell. 1993. GenBank. Nucleic Acids Res. **21:**2963-2965.
6. Biavati, B., B. Sgorbati, and V. Scardovi. 1991. The genus *Bifidobacterium*, p. 816-833. *In* ., A. Balows, H. G. Trüper, M. Dworkin, W. Harder, and K. H. Schleifer (ed.), The prokaryotes, 2nd ed. Springer-Verlag, New York.
7. Biavati, B., P. Martelli, and F. Crociani. 1992. Identification of bifidobacteria from fermented milk products. Microbiologica **15:**7-14.
8. Brosius, J., T. J. Dull, D. D. Sleeter, and H. F. Noller. 1981. Gene oragnization and primary structure of a ribosomal RNA operon from *Escherichia coli*. J. Mol. Biol. **148:**107-127.
9. Brunk, C. F., E. Avaniss-Aghajyni, and C. A. Brunk. 1996. A computer analysis of primer and probe hybridization potential with bacterial small-subunit rRNA sequences. Appl. Environ. Microbiol. **62:**872-879.
10. Carrino, J. J., and H. H. Lee. 1995. Nucleic acid amplification methods. J. Microbiol. Methods **23:**3-20.
11. Crociani, F., B. Biavati, A. Alessandrini, C. Chiarini, and V. Scardovi. 1996. *Bifidobacterium inopinatum* sp. nov. and *Bifidobacterium denticolens* sp. nov., two new species isolated from human dental caries. Int. J. Syst. Bacteriol. **2:**564-571.
12. Embley, T. M., and E. Stackebrandt. 1994. The molecular phylogeny and systematics of the actinomycetes. Annu. Rev. Microbiol. **48:**257-289.
13. Feinberg, A. P., and B. Vogelstein. 1983. A technique for radiolabelling DNA restriction endonuclease fragments to high specific activity. Anal. Biochem. **132:**6-13.
14. Frothingham, R., A. J. Duncan, and K. H. Wilson. 1993. Ribosomal DNA sequences of bifidobacteria: Implications for sequence-based identification of the human colonic flora. Microb. Ecol. Health Dis. **6:**23-27.
15. Gavini, F., A.-M. Pourcher, C. Neut, D. Monget, C. Romond, C. Oger, and D. Izard. 1991. Phenotypic differentiation of Bifidobacteria of human and animal origins. Int. J. Syst. Bacteriol. **41:**548-557.
16. Gendel, S. M. 1996. Computational analysis of the specificity of 16S rRNA-derived signature sequences for identifying food-related microbes. Food Microbiol. **13:**1-15.
17. Ghoddusi, H. B., and R. K. Robinson. 1996. Enuneration of starter cultures in fermented milks. J. Dairy Res. **63:**151-158.
18. Gibson, G. R., and M. B. Roberfroid. 1995. Dietary modulation of the human colonic microbiota: introducing the concept of prebiotics. J. Nutr. **6:**1401-1412.
19. Goldenberger, D., I. Perschil, M. Ritzler, and M. Altwegg. 1995. A simple 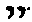 universal 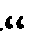 DNA extraction procedure using SDS and proteinase K is compatible with direct PCR amplification. PCR Meth. Appl. **4:**368-370.
20. Greenwood, J. R., and M. J. Picket. 1986. The genus *Gardnerella*. p. 1283-1286. *In* P. H. A. Sneath, N. S. Mair, M. E. Sharpe, and J. G. Holt (ed.), Bergey's manual of systematic bacteriology, vol. 2. Williams and Wilkins, Baltimore.
21. Grill, J. P., C. Manginot-Dürr, F. Schneider, and J. Ballongue. 1995. Bifidobacteria and probiotic effects: action of *Bifidobacterium* species on conjugated bile salts. Curr. Microbiol. **31:**23-27.
22. Langendijk, P. S., F. Schut, G. J. Jansen, G. C. Raangs, G. R. Kamphuis, M. H. F. Wilkinson, and G. W. Welling. 1995. Qunatitative fluorescence *in situ* hybridization of *Bifidobacterium* ssp. with genus-specific 16s rRNA-targeted probes and its application in fecal samples. Appl. Environ. Microbiol. **61:**3069-3075.
23. Lapierre, L., P. Undeland, and L. J. Cox. 1992. Lithium chloride - sodium propionate agar for the enumeration of bifidobacteria in fermented dairy products. J. Dairy Sci. **75:**1192-1196.
24. Larsen, N., G. J. Olsen, B. L. Maidak, M, J. Mccaughey, R. Overbeek, T. J. Macke, T. L. Marsh, and C. R. Woese. 1993. The ribosomal database project. Nucleic Acids Res. **21:**3021-3023.
25. Leblond-Bourget N., H. Philippe, I. Mangin, and B. Decaris. 1996. 16S rRNA and 16S to 23S internal transcribed spacer sequence analyses reveal inter- and intraspecific *Bifidobacterium* phylogeny. Int. J. Syst. Bacteriol. **46:**102-111.
26. Lee, Y.-K., and S. Salminen. 1995. The coming age of probiotics. Trends Food Sci. & Technol. **6:**241-245.
27. Ludwig, W. and K. H. Schleifer. 1994. Bacterial phylogeny based on 16S and 23S rRNA sequences analysis. FEMS Microbiol. Lett. **15:**155-173.
28. Mangin, I., N. Bourget, and B. Decaris. 1996. Ribosomal DNA polymorphism in the genus *Bifidobacterium*. Res. Microbiol. **147:**183-192.
29. Mangin, I., N. Bourget, J.-M. Simonet, and B. Decaris. 1995. Selection of species-specific DNA probes which detect strain restriction polymorphism in four *Bifidobacterium* species. Res. Microbiol. **146:**59-71.
30. Neefs, J.-M., Y. Van der Peer, P. De Rijk, S. Chapelle, and R. de Wachter. 1993. Compilation of small ribosomal subunit RNA structures. Nucleic Acids Res. **21:**3025-3049.
31. Olsen, G. H., C. R. Woese, and R. Overbeek. 1994. The winds of (evolutionary) change: breathing new life into microbiology. J. Bacteriol. **176:**1-6.
32. Rice, C. M., R. Fuchs, D. G. Higgins, P. J. Stoehr, and G. N. Cameron. 1993. The EMBL data library. Nucleic Acids Res. **21:**2967-2971.
33. Rijpens, N. P., G. Jannes, M. van Asbroeck, R. Rossau, and L. M. F. Herman. 1996. Direct detection of *Brucella* ssp. in raw milk by PCR and reverse hybridization with 16S-23S rRNA spacer probes. Appl. Environ. Microbiol. **5:**1683-1688.
34. Roy, D., M.-L. Desjardins, and F. Mondou. 1995. Selection of Bifidobacteria for use under cheese-making conditions. Milchwissenschaft **3:**139-142.
35. Sambrook, J., E. F. Fritsch, and T. Maniatis. 1989. Molecular cloning, a laboratory manual. 2nd ed., Cold Spring Harbor Laboratory Press, New York.
36. Schleifer, K. H. and W. Ludwig. 1995. Phylogenetic relationships of lactic acid bacteria, p. 7-18. *In* B. J. B. Wood and W. H. Holzapfel (ed), The genera of lactic acid bacteria. Blacky Academic & Professional, London.
37. Sgorbati, B., B. Biavati, and D. Palenzona. 1995. The genus *Bifidobacterium*, p. 279-306. *In* B. J. B. Wood and W. H. Holzapfel (ed), The genera of lactic acid bacteria. Blacky Academic & Professional, London.
38. Stackebrandt, E., and B. M. Goebel. 1994. Taxonomic note: a place for DNA-DNA reassociation and 16S rRNA sequencing analysis in the present species definition in bacteriology. Int. J. Syst. Bacteriol. **44:**846-849.
39. Wang, R.-F., W.-W. Cao, and C. E. Cerniglia. 1996. PCR detection and quantitation of predominant anaerobic bacteria in human and animal fecal samples. Appl. Environ. Microbiol. **4:**1242-1247.
40. Yamamoto, T., M. Morotomi, and R. Tanaka. 1992. Species-specific oligonucleotide probes for five *Bifidobacterium* species detected in human intestinal microflora. Appl. Environ. Microbiol. **58:**4076-4079.

## Claims

**1.** A new oligonucleotide probe for the specific detection of bacteria of the genus *Bifidobacterium* comprising the sequence 5'-CGGGTGCTNCCACTTTCATG-3', Seq Id No 1, wherein A,T,C and G have their regular meanings for DNA, adenine, thymine, cytosine and guanine, respectively, and N represents inosine or another chemical hybridizing to all bases occurring at this position of the DNA or RNA bifidobacteria target, a fragment thereof, the complementary sequence thereof, or a labelled derivative thereof.

**2.** An oligonucleotide according to claim 1 having the sequence 5'-CGGGTGCTNCCCACTTTCATG-3', Seq Id No 1, wherein N represents inosine.

**3.** An oligonucleotide according to claim 1 which is labelled at one or several nucleotides by chemical, fluorescent or radioactive labels which are detectable after hybridization with the target by the approriate analytical method.

**3.** Use of an oligonucleotide according to claim 1 for the detection and identification of bacteria of the genus *Bifidobacterium*.

**4.** A method for the detection of the bacteria of the genus *Bifidobacterium* comprising targeting an oligonucleotide according to claim 1 to the 16S rDNA or 16S rRNA of said bacterium.

**5.** A method according to claim 4, in which the detection is based on a polymerase chain reaction technique by means of an oligonucleotide according to claim 1 and a primer comprising the sequence 5'-GATTCTGGCTCAGGATGAACG-3', Seq Id No 2, or a hybridizing fragment thereof.

**6.** A method according to claim 4, in which the detection is based on a dot-blot assay, where the DNA or RNA target is fixed on a nylon or another suitable membrane.

**7.** A method according to claim 4, in which the bacteria are serially diluted and streaked out on agar-plates.

**8.** A method according to claim 4, in which the bacteria are detected in smples of food, feces or any other environment.

**9.** An analytical kit for the qualitative and quantitative detection of bacteria of the genus *Bifidobacterium*, comprising an oligonucleotide according to claim 1, optionally a second oligonucleotide primer according to claim 5 and optionally other conventional materials needed for the qualitative or quantitative detection of the bacteria of the genus *Bifidobacterium*.


SEQUENCE LISTING

(1) GENERAL INFORMATION:

(i) APPLICANT:

(A) NAME: Kaufmann, Peter

(B) STREET: Schmelzbergstr. 9

(C) CITY: Zuerich

(E) COUNTRY: CH

(F) POSTAL CODE (ZIP): CH-8092

(G) TELEPHONE: xx41-1-6323334

(H) TELEFAX: xx41-1-6321266

(A) NAME: Meile, Leo

(B) STREET: Schmelzbergstr. 9

(C) CITY: Zuerich

(E) COUNTRY: CH

(F) POSTAL CODE (ZIP): CH-8092

(G) TELEPHONE: xx41-1-6323334

(H) TELEFAX: xx41-1-6321266

(A) NAME: Teuber, Michael

(B) STREET: Schmelzbergstr.9

(C) CITY: Zuerich

(E) COUNTRY: CH

(F) POSTAL CODE (ZIP): CH-8092

(G) TELEPHONE: xx41-1-6323334

(H) TELEFAX: xx41-1-6321266

(ii) TITLE OF INVENTION: Oligonucleotide Probes for the
Detection of Bifidobacteria

(iii) NUMBER OF SEQUENCES: 3

(iv) COMPUTER READABLE FORM:

(A) MEDIUM TYPE: Floppy disk

(B) COMPUTER: IBM PC compatible

(C) OPERATING SYSTEM: PC-DOS/MS-DOS

(D) SOFTWARE: PatentIn Release #1.0, Version #1.30
(EPO)
(2) INFORMATION FOR SEQ ID NO: 1:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: other nucleic acid
(A) DESCRIPTION: /desc = "Synthetic nucleic acid"
(iii) HYPOTHETICAL: NO
(iv) ANTI-SENSE: NO
(v) FRAGMENT TYPE: internal
(viii) POSITION IN GENOME:
(A) CHROMOSOME/SEGMENT: V9 region in 16S rRNA
(B) MAP POSITION: 1412 - 1432(E.coli numbering,
Brosius)
(C) UNITS: 21 bp
(ix) FEATURE:
(A) NAME/KEY: primer_bind
(B) LOCATION:complement (1..21)
(ix) FEATURE:
(A) NAME/KEY: misc_binding
(B) LOCATION:complement (1..21)
(xi) SEQUENCE DESCRIPTION: SEQ ID NO: 1:
CGGGTGCTNC CCACTTTCAT G 21

(2) INFORMATION FOR SEQ ID NO: 2:

(i) SEQUENCE CHARACTERISTICS:

(A) LENGTH: 21 base pairs

(B) TYPE: nucleic acid

(C) STRANDEDNESS: single

(D) TOPOLOGY: linear

(ii) MOLECULE TYPE: other nucleic acid

(A) DESCRIPTION: /desc = "Synthetic nucleic acid"

(iii) HYPOTHETICAL: NO

(iv) ANTI-SENSE: NO

(v) FRAGMENT TYPE: internal

(viii) POSITION IN GENOME:

(A) CHROMOSOME/SEGMENT: S16 rRNA

(B) MAP POSITION: 15-35 (E. coli numbering, Brosius)

(C) UNITS: 21 bp

(ix) FEATURE:

(A) NAME/KEY: primer_bind

(B) LOCATION:complement (1..21)

(ix) FEATURE:

(A) NAME/KEY: misc_binding

(B) LOCATION:complement (1..21)

(xi) SEQUENCE DESCRIPTION: SEQ ID NO: 2:

GATTCTGGCT CAGGATGAAC G 21

(2) INFORMATION FOR SEQ ID NO: 3:

(i) SEQUENCE CHARACTERISTICS:

(A) LENGTH: 21 base pairs

(B) TYPE: nucleic acid

(C) STRANDEDNESS: single

(D) TOPOLOGY: linear

(ii) MOLECULE TYPE: other nucleic acid

(A) DESCRIPTION: /desc = "synthetic"

(iii) HYPOTHETICAL: NO

(iv) ANTI-SENSE: NO

(v) FRAGMENT TYPE: internal

(viii) POSITION IN GENOME:

(B) MAP POSITION: 1412-1432(E.coli numbering,Brosius)

(C) UNITS: 21 bp

(ix) FEATURE:

(A) NAME/KEY: misc_binding

(B) LOCATION:complement (1..21)

(xi) SEQUENCE DESCRIPTION: SEQ ID NO: 3:

CATGAAAGTG GGNAGCACCC G 21